(19) Europäisches Patentamt
European Patent Office
Office européen des brevets

(11) EP 1 569 909 B1

# (12) EUROPÄISCHE PATENTSCHRIFT

(45) Veröffentlichungstag und Bekanntmachung des Hinweises auf die Patenterteilung:
**05.07.2006 Patentblatt 2006/27**

(21) Anmeldenummer: **03812587.8**

(22) Anmeldetag: **22.11.2003**

(51) Int Cl.:
***C07D 231/38*** (2006.01) ***A01N 43/56*** (2006.01)

(86) Internationale Anmeldenummer:
**PCT/EP2003/013141**

(87) Internationale Veröffentlichungsnummer:
**WO 2004/052865 (24.06.2004 Gazette 2004/26)**

## (54) SUBSTITUIERTE PYRAZOLINCARBOXANILIDE ALS SCHÄDLINGSBEKÄMPFUNGMITTEL

SUBSTITUTED PYRAZOLINE CARBOXANILIDES FOR USE AS PESTICIDES

PYRAZOLINE-CARBOXANILIDES SUBSTITUES UTILISES COMME PESTICIDES

(84) Benannte Vertragsstaaten:
**AT BE BG CH CY CZ DE DK EE ES FI FR GB GR HU IE IT LI LU MC NL PT RO SE SI SK TR**

(30) Priorität: **06.12.2002 DE 10257080**

(43) Veröffentlichungstag der Anmeldung:
**07.09.2005 Patentblatt 2005/36**

(73) Patentinhaber: **Bayer CropScience AG**
**40789 Monheim (DE)**

(72) Erfinder:
- **FUCHS, Rainer**
  **42113 Wuppertal (DE)**
- **MAURER, Fritz**
  **74348 Lauffen a.N. (DE)**
- **KONZE, Jörg**
  **51147 Köln (DE)**
- **ARNOLD, Christian**
  **40764 Langenfeld (DE)**

(56) Entgegenhaltungen:
**EP-A- 0 438 690**
**EP-A- 0 679 644**
**DE-A- 10 135 551**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 569 909 B1

Printed by Jouve, 75001 PARIS (FR)

## Beschreibung

Die vorliegende Erfindung betrifft neue substituierte Pyrazolincarboxanilide, Verfahren zu ihrer Herstellung und ihre Verwendung als Schädlingsbekämpfungsmittel, insbesondere als Arthropodizide.

Es ist bekannt, dass bestimmte substituierte Pyrazolincarboxanilide insektizide und akarizide Eigenschaften aufweisen (vgl. z.B. EP-A 0 438 690, EP-A 0 679 644). Die Wirkung dieser Verbindungen ist jedoch, insbesondere bei niedrigen Wirkstoffkonzentrationen und Aufwandmengen nicht immer ganz befriedigend.

Es wurden neue substituierte Pyrazolincarboxanilide der Formel (I) gefunden,

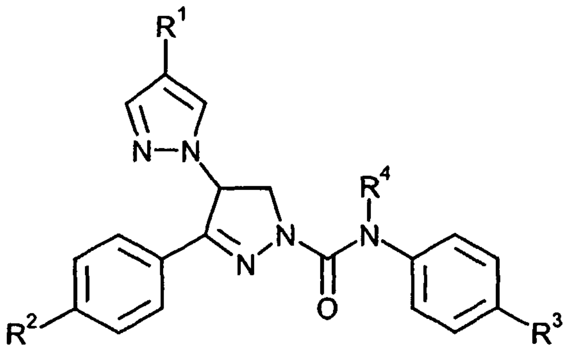

(I)

in welcher

$R^1$ für Halogen steht,
$R^2$ für Cyano, Halogen, Halogenalkyl, Halogenalkoxy, Alkylthio, Halogenalkylthio, Alkylsulfinyl, Halogenalkylsulfinyl, Alkylsulfonyl oder Halogenalkylsulfonyl steht,
$R^3$ für Cyano, Halogen, Halogenalkyl, Halogenalkoxy, Alkylthio, Halogenalkylthio, Alkylsulfinyl, Halogenalkylsulfmyl, Alkylsulfonyl oder Halogenalkylsulfonyl steht und
$R^4$ für Cyanoalkyl steht.

Die Verbindungen der Formel (I) können gegebenenfalls in Abhängigkeit der Art und Anzahl der Substituenten als geometrische und/oder optische Isomere, Regioisomere bzw. Konfigurationsisomere oder deren Isomerengemische in unterschiedlicher Zusammensetzung vorliegen. Sowohl die reinen Isomeren als auch die Isomerengemische werden erfindungsgemäß beansprucht.

Weiterhin wurde gefunden, dass man die substituierten Pyrazolincarboxanilide der Formel (I) erhält, indem man

(a) substituierte Pyrazoline der Formel (II)

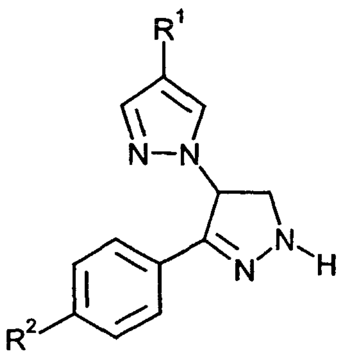

(II)

in welcher $R^1$ und $R^2$ die oben angegebenen Bedeutungen haben,
mit Isocyanaten der Formel (III)

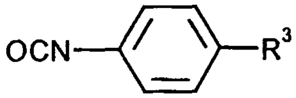

(III)

in welcher $R^3$ die oben angegebenen Bedeutungen hat,
gegebenenfalls in Gegenwart eines Verdünnungsmittels und gegebenenfalls in Gegenwart eines Katalysators umsetzt und die so erhaltenen substituierten Pyrazolincarboxanilide der Formel (Ia)

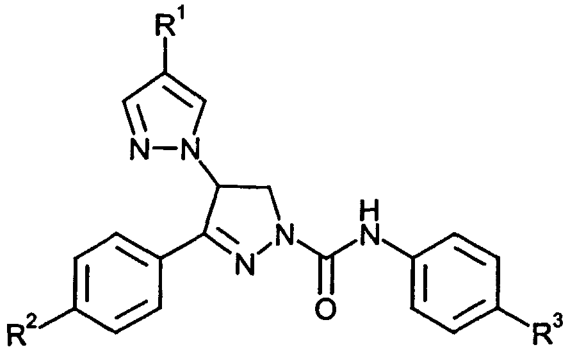

(Ia)

in welcher $R^1$, $R^2$ und $R^3$ die oben angegebenen Bedeutungen haben,
mit Halogenverbindungen der Formel (IV)

$X^1$-$R^4$ (IV)

in welcher

$R^4$ die oben angegebene Bedeutung hat und
$X^1$ für Halogen steht,

gegebenenfalls in Gegenwart eines Verdünnungsmittels und gegebenenfalls in Gegenwart einer Base umsetzt,
oder dass man die substituierten Pyrazolincarboxanilide der Formel (Ib)

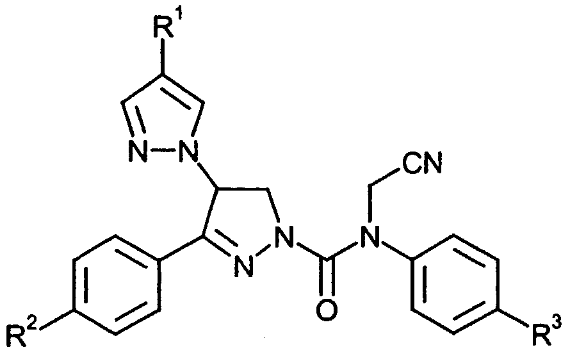

(Ib)

in welcher R', $R^2$ und $R^3$ die oben angegebenen Bedeutungen haben, erhält, indem man
(b) substituierte Pyrazoline der Formel (II)

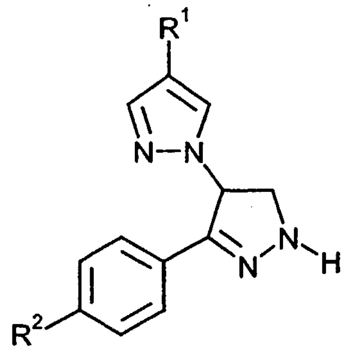

(II)

in welcher $R^1$ und $R^2$ die oben angegebenen Bedeutungen haben,
mit Carbamidsäurechloriden der Formel (V)

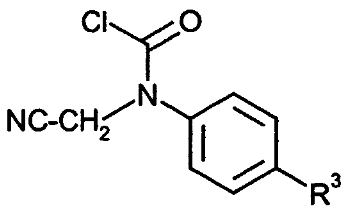

(V)

in welcher $R^3$ die oben angegebenen Bedeutungen hat,
in Gegenwart eines Verdünnungsmittels und gegebenenfalls in Gegenwart einer Base umsetzt.

Schließlich wurde gefunden, dass die neuen substituierten Pyrazolincarboxanilide der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in Forsten, im Vorrats- und Materialschutz sowie auf dem Hygienesektor vorkommen, geeignet sind.

Die erfindungsgemäßen substituierten Pyrazolincarboxanilide sind durch die Formel (I) allgemein definiert.

Bevorzugte Substituenten bzw. Bereiche der in den oben und nachstehend erwähnten Formeln aufgeführten Reste werden im folgenden erläutert:

$R^1$ steht bevorzugt für Chlor, Brom oder Iod.

$R^1$ steht besonders bevorzugt für Chlor oder Brom.

$R^2$ steht bevorzugt für Cyano, Fluor, Chlor, Brom, Iod, $C_1$-$C_4$-Halogenalkyl, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl oder $C_1$-$C_4$-Halogenalkylsulfonyl.

$R^2$ steht besonders bevorzugt für Cyano, Fluor, Chlor, Brom, Iod, Monofluormethyl, Difluormethyl, Trifluormethyl, Monochlormethyl, Dichlormethyl, Trichlormethyl, Chlordifluormethyl, Fluordichlormethyl, Monofluorethyl, Difluorethyl, Trifluorethyl, Tetrafluorethyl, Pentafluorethyl, Monochlorethyl, Dichlorethyl, Trichlorethyl, Chlorfluorethyl, Chlordifluorethyl, Chlortrifluorethyl, Fluordichlorethyl, Dichlordifluorethyl, Difluormethoxy, Trifluormethoxy, Chlordifluormethoxy, Fluorethoxy, Difluorethoxy, Trifluorethoxy, Tetrafluorethoxy, Chlorethoxy, Dichlorethoxy, Chlorfluorethoxy, Methylthio, Ethylthio, n- oder i-Propylthio, Difluormethylthio, Trifluormethylthio, Chlordifluormethylthio, Methylsulfinyl, Ethylsulfinyl, Difluormethylsulfinyl, Trifluormethylsulfinyl, Methylsulfonyl, Ethylsulfonyl, Difluormethylsulfonyl oder Trifluormethylsulfonyl.

$R^2$ steht ganz besonders bevorzugt für Cyano, Fluor, Chlor, Brom, Iod, Difluormethyl, Trifluormethyl, Dichlormethyl, Trichlormethyl, Chlordifluormethyl, Fluordichlormethyl, Difluormethoxy, Trifluormethoxy, Chlordifluormethoxy, Difluormethylthio, Trifluormethylthio, Chlordifluormethylthio, Trifluormethylsulfinyl oder Trifluormethylsulfonyl.

$R^3$ steht bevorzugt für Cyano, Fluor, Chlor, Brom, Iod, $C_1$-$C_4$-Halogenalkyl, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl oder $C_1$-$C_4$-Halogenalkylsulfonyl.

$R^3$ steht besonders bevorzugt für Cyano, Fluor, Chlor, Brom, Iod, Monofluormethyl, Difluormethyl, Trichlormethyl, Monochlormethyl, Dichlormethyl, Trichlormethyl, Chlordifluormethyl, Fluordichlormethyl, Monofluorethyl, Difluorethyl, Trifluorethyl, Tetrafluorethyl, Pentafluorethyl, Monochlorethyl, Dichlorethyl, Trichlorethyl, Chlorfluorethyl, Chlordifluorethyl, Chlortrifluorethyl, Fluordichlorethyl, Dichlordifluorethyl, Difluormethoxy, Trifluormethoxy, Chlordifluormethoxy, Fluorethoxy, Difluorethoxy, Trifluorethoxy, Tetrafluorethoxy, Chlorethoxy, Dichlorethoxy, Chlorfluorethoxy, Methylthio, Ethylthio, n- oder i-Propylthio, Difluormethylthio, Trifluormethylthio, Chlordifluormethylthio, Methylsulfinyl, Ethylsulfinyl, Difluormethylsulfinyl, Trifluormethylsulfinyl, Methylsulfonyl, Ethylsulfonyl, Difluormethylsulfonyl oder Trifluormethylsulfonyl.

$R^3$ steht ganz besonders bevorzugt für Cyano, Fluor, Chlor, Brom, Iod, Difluormethyl, Trifluormethyl, Dichlormethyl, Trichlormethyl, Chlordifluormethyl, Fluordichlormethyl, Difluormethoxy, Trifluormethoxy, Chlordifluormethoxy, Difluormethylthio, Trifluormethylthio, Chlordifluormethylthio, Trifluormethylsulfinyl oder Trifluormethylsulfonyl.

$R^4$ steht bevorzugt für Cyano-$C_1$-$C_4$-alkyl.

$R^4$ steht besonders bevorzugt für Cyanomethyl, Cyanoethyl oder Cyanopropyl.

$R^4$ steht ganz besonders bevorzugt für Cyanomethyl.

Insbesondere bevorzugt sind Verbindungen der Formel (I), in welcher $R^1$ für Chlor steht.
Insbesondere bevorzugt sind Verbindungen der Formel (I), in welcher $R^4$ für Cyanomethyl steht.
Insbesondere bevorzugt sind Verbindungen der Formel (I), in welcher $R^1$ für Chlor und $R^4$ für Cyanomethyl steht.
Insbesondere bevorzugt sind Verbindungen der Formel (I), in welcher $R^2$ für Fluor, Chlor oder Brom, besonders bevorzugt für Chlor steht.
Insbesondere bevorzugt sind Verbindungen der Formel (I), in welcher $R^3$ für Cyano, Chlor, Brom, Trifluormethyl, Trifluormethoxy oder Trifluormethylthio steht.

Die oben aufgeführten oder in Vorzugsbereichen aufgeführten Restedefinitionen bzw. Erläuterungen gelten für die Endprodukte und für die Ausgangs- und Zwischenprodukte entsprechend. Diese Restedefinitionen können untereinander, also auch zwischen den jeweiligen Vorzugsbereichen, beliebig kombiniert werden.

Erfindungsgemäß bevorzugt werden die Verbindungen der Formel (I), in welchen eine Kombination der vorstehend als bevorzugt aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.
Erfindungsgemäß ganz besonders bevorzugt werden die Verbindungen der Formel (I), in welchen eine Kombination der vorstehend als ganz besonders bevorzugt aufgeführten Bedeutungen vorliegt.

In den oben und nachstehend aufgeführten Restedefinitionen sind Kohlenstoffreste, wie Alkyl - auch in Verbindung mit Heteroatomen wie Alkoxy - soweit möglich jeweils geradkettig oder verzweigt.

Durch Halogen substituierte Reste, wie z.B. Halogenalkyl, sind einfach oder mehrfach halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Verwendet man beispielsweise 3-(4-Chlor-phenyl)-4-(4-brom-pyrazol-l-yl)-4,5-dihydro-1H-pyrazol und 4-Chlor-phenylisocyanat sowie in der Folgestufe Bromacetonitril als Ausgangsstoffe, so kann der Reaktionsablauf des erfindungsgemäßen Verfahrens (a) durch das folgende Formelschema wiedergegeben werden:

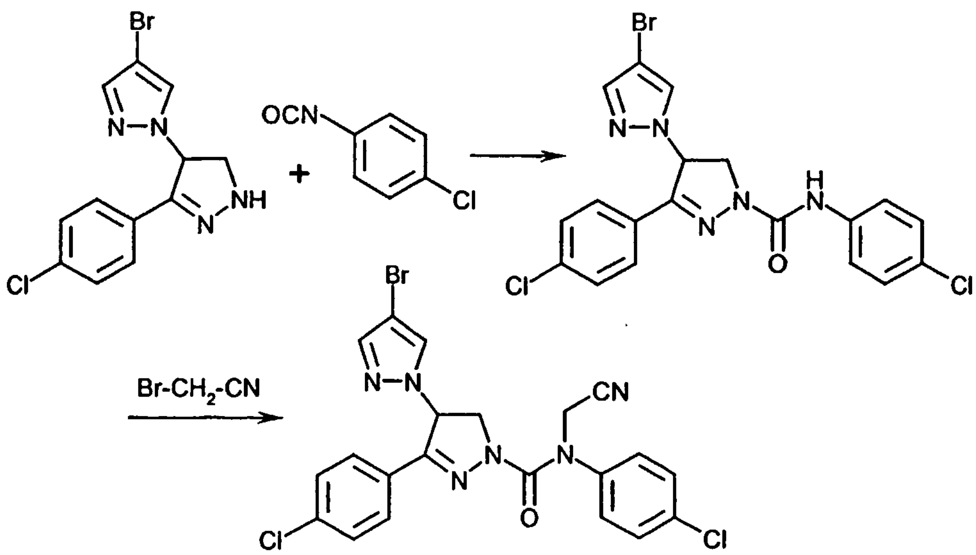

Verwendet man beispielsweise 3-(4-Chlor-phenyl)-4-(4-chlor-pyrazol-1-yl)-4,5-dihydro-1H-pyrazol und N-Cyanomethyl-N-(4-trifluormethoxy-phenyl)-carbamidsäurechlorid als Ausgangsstoffe, so kann der Reaktionsablauf des erfindungsgemäßen Verfahrens (b) durch das folgende Formelschema wiedergegeben werden:

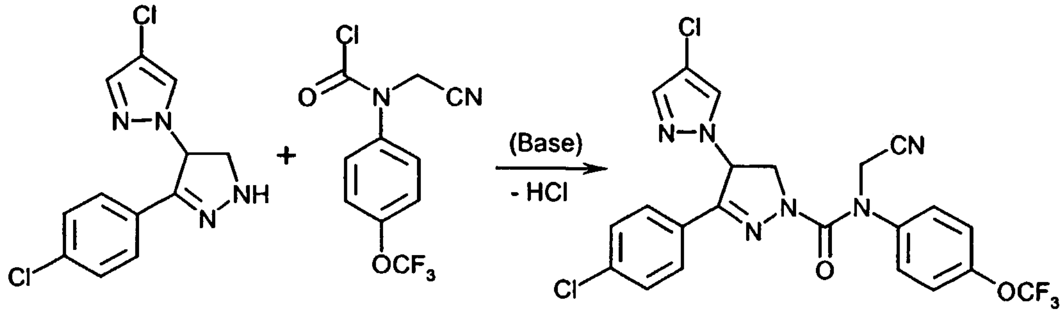


Die zur Durchführung der erfindungsgemäßen Verfahren (a) und (b) als Ausgangsstoffe zu verwendenden substituierten Pyrazoline sind durch die Formel (II) allgemein definiert. In dieser Formel stehen $R^1$ und $R^2$ bevorzugt, besonders bevorzugt bzw. ganz besonders bevorzugt für diejenigen Bedeutungen, die bereits in Zusammenhang mit der Beschreibung der erfindungsgemäßen Verbindungen der Formel (I) für diese Reste als bevorzugt, besonders bevorzugt bzw. ganz besonders bevorzugt genannt wurden.

Die substituierten Pyrazoline der Formel (II) sind bekannt und/oder können nach an sich bekannten Verfahren hergestellt werden (vgl. EP-A 0 438 690, EP-A 0 546 420 und die Herstellungsbeispiele).

Die außerdem beim erfindungsgemäßen Verfahren (a) als Ausgangsstoffe zu verwendenden Isocyanate sind durch die Formel (III) allgemein definiert. In dieser Formel steht $R^3$ bevorzugt, besonders bevorzugt bzw. ganz besonders bevorzugt für diejenigen Bedeutungen, die bereits in Zusammenhang mit der Beschreibung der erfindungsgemäßen Verbindungen der Formel (I) für diesen Rest als bevorzugt, besonders bevorzugt bzw. ganz besonders bevorzugt genannt wurden.

Die Isocyanate der Formel (III) sind allgemein bekannte Verbindungen der organischen Chemie und/oder können in allgemein bekannter Art und Weise erhalten werden.

Die zur Durchführung des erfindungsgemäßen Verfahrens (a) weiter als Ausgangsstoffe zu verwendenden Halogenverbindungen sind durch die Formel (IV) allgemein definiert. In dieser Formel steht $R^4$ bevorzugt, besonders bevorzugt bzw. ganz besonders bevorzugt für diejenigen Bedeutungen, die bereits in Zusammenhang mit der Beschreibung der erfindungsgemäßen Verbindungen der Formel (I) für diesen Rest als bevorzugt, besonders bevorzugt bzw. ganz besonders bevorzugt genannt wurden. $X^1$ steht bevorzugt für Chlor oder Brom.

Die Halogenverbindungen der Formel (IV) sind allgemein bekannte Verbindungen der organischen Chemie.

Die außerdem beim erfindungsgemäßen Verfahren (b) als Ausgangsstoffe zu verwendenden Carbamidsäurechloride sind durch die Formel (V) allgemein definiert. In dieser Formel steht $R^3$ bevorzugt, besonders bevorzugt bzw. ganz besonders bevorzugt für diejenigen Bedeutungen, die bereits im Zusammenhang mit der Beschreibung der erfindungsgemäßen Verbindungen der Formel (I) für diesen Rest als bevorzugt, besonders bevorzugt bzw. ganz besonders bevorzugt genannt wurden.

Die Carbamidsäurechloride der Formel (V) sind bekannt und/oder können nach an sich bekannten Verfahren hergestellt werden (vg1. DE-A 27 30 325, DE-A 101 35 551 vom 20. Juli 2001 und die Herstellungsbeispiele).

Das erfindungsgemäße Verfahren (a) wird vorzugsweise unter Verwendung eines oder mehrerer Verdünnungsmittel durchgeführt. Als Verdünnungsmittel kommen praktisch alle inerten organischen Lösungsmittel in Betracht. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlormethan, Chlorbenzol und o-Dichlorbenzol, Ether wie Diethyl-, Di-isopropyl- und Di-isobutylether, Methyl-tert.-butyl-ether, Methyl-tert.-amylether, Glykoldimethylether und Diglykoldimethylether, Tetrahydrofuran und Dioxan, Ketone wie Aceton, Methyl-ethyl-keton, Methyl-isopropyl-keton oder Methyl-isobutyl-keton, Ester wie Essigsäuremethylester oder -ethylester, Nitrile wie Acetonitril oder Propionitril, Amide wie N,N-Dimethyl-formamid, N,N-Dimethylacetamid und N-Methyl-pyrrolidon sowie Dimethylsulfoxid, Tetramethylensulfon oder Hexamethylphosphorsäuretriamid.

Das erfindungsgemäße Verfahren (a) wird vorzugsweise auch unter Verwendung eines Katalysators durchgeführt. Als Katalysatoren kommen dabei insbesondere tertiäre organische Amine, wie beispielsweise Triethylamin in Betracht.

Als Basen können bei der Durchführung der erfindungsgemäßen Verfahren (a) und (b) alle üblicherweise für derartige Umsetzungen verwendbaren Säurebindemittel eingesetzt werden. Vorzugsweise in Frage kommen Alkalimetall- und Erdalkalimetall-hydride, wie Lithium-, Natrium-, Kalium- oder Calciumhydrid; Alkalimetall- und Erdalkalimetall-hydroxide, wie Lithium-, Natrium-, Kalium- oder Calciumhydroxid; Alkalimetall- und Erdalkalimetallcarbonate oder -hydrogencarbonate, wie Natrium- oder Kaliumcarbonat oder -hydrogencarbonat oder Calciumcarbonat; Alkalimetallacetate, wie Natrium- oder Kaliumacetat, Alkalimetallalkoholate, wie Natrium- oder Kalium-tert.-butylat; ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethylanilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo [4.3.0]-non-5-en (DBN), 1,8-Diazabicyclo[5.4.0]-undec-7-en (DBU), 1,4-Diazabicyclo[2.2.2]-octan (DABCO).

Die Reaktionstemperaturen können beim erfindungsgemäßen Verfahren (a) in einem größeren Bereich variiert werden. Im allgemeinen arbeitet man bei Temperaturen zwischen 0°C und 120°C, vorzugsweise bei Temperaturen zwischen 20°C und 80°C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die Ausgangsstoffe im allgemeinen in angenähert äquimolaren Mengen eingesetzt. Es ist jedoch auch möglich, eine der beiden eingesetzten Komponenten in einem kleineren Überschuss zu verwenden. Die Aufarbeitung erfolgt nach üblichen Methoden (vg1. die Herstellungsbeispiele).

Das erfindungsgemäße Verfahren (b) wird vorzugsweise unter Verwendung eines oder mehrerer Verdünnungsmittel durchgeführt. Als Verdünnungsmittel kommen praktisch alle inerten organischen Lösungsmittel in Betracht. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-, Di-isopropyl- und Di-tert.-butyl-ether, Glykoldimethylether und Diglykoldimethylether, Tetrahydrofuran und Dioxan, Ketone wie Aceton, Methyl-ethyl-, Methyl-isopropyl- oder Methyl-isobutyl-keton, Ester wie Essigsäuremethylester oder -ethylester, Nitrile wie z.B. Acetonitril oder Propionitril, Amide wie z.B. N,N-Dimethyl-formamid, N,N-Dimethyl-acetamid und N-Methyl-pyrrolidon sowie Dimethylsulfoxid, Tetramethylensulfon oder Hexamethylenphosphorsäuretriamid.

Die Reaktionstemperaturen können beim erfindungsgemäßen Verfahren (b) in einem größeren Bereich variiert werden. Im allgemeinen arbeitet man bei Temperaturen zwischen - 20°C und 120°C, bevorzugt bei Temperaturen zwischen 0°C und 80°C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die Ausgangsstoffe im allgemeinen in angenähert äquimolaren Mengen eingesetzt. Es ist jedoch auch möglich, das Halogenid und die Base im Überschuss zu verwenden. Die Aufarbeitung erfolgt nach üblichen Methoden (vgl. die Herstellungsbeispiele).

Die Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Emtegutes und zur Bekämpfung von tierischen Schädlingen, insbesondere Insekten, Spinnentieren und Nematoden, die in der Landwirtschaft, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica.
Aus der Ordnung der Dermaptera z.B. Forficula auricularia.
Aus der Ordnung der Isoptera z.B. Reticulitermes spp.
Aus der Ordnung der Phthiraptera z.B. Pediculus humanus corporis, Haematopinus spp., Linognathus spp., Trichodectes spp., Damalinia spp.
Aus der Ordnung der Thysanoptera z.B. Hercinothrips femoralis, Thrips tabaci, Thrips palmi, Frankliniella occidentalis.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogöderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp.

Aus der Ordnung der Siphonaptera z.B. Xenopsylla cheopis, Ceratophyllus spp.

Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp.

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp.

Die erfindungsgemäßen Verbindungen der Formel (I) zeichnen sich insbesondere durch sehr starke Wirksamkeit gegen Larven des Meerrettichblattkäfers (Phaedon cochleariae), gegen Raupen der Kohlschabe (Plutella xylostella), gegen Raupen des Heerwurms (Spodoptera frugiperda) und gegen Raupen des Baumwollkapselwurms (Heliothis armigera) aus.

Die erfindungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Samen, weiterhin durch ein- oder mehrschichtiges Umhüllen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

**Fungizide:**

2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesüate); Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrin; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4- chlorophenyl)- 2- propynyl] oxy]- 3- methoxyphenyl) ethyl]- 3- methyl- 2-[(methylsulfonyl) amino]-butanamide; 1-(1-naphthalenyl)-1H-pyrrole-2,5-dione; 2,3,5,6-tetrachloro-4-(methylsulfonyl)-pyridine; 2-amino-4-methyl-N-phenyl-5-thiazolecarboxamide; 2-chloro-N-(2,3-dihydro-1,1,3-trimethyl-1H-inden-4-yl)-3-pyridincarboxamide; 3,4,5-trichloro-2,6-pyridinedicarbonitrile; Actinovate; cis-1-(4-chlorophenyl)-2-(1H-1,2,4-triazole-1-yl)-cyloheptanol; methyl 1-(2,3-dihydro-2,2-dimethyl-1H-inden-1-yl)-1H-imidazole-5-carboxylate; monopotassium carbonate; N-(6-methoxy-3-pyridinyl)-cyclopropanecarboxamide; N-butyl-8-(1,1-dimethylethyl)-1-oxaspiro[4.5]decan-3-amine; Sodium tetrathiocarbonate;
sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxine-copper.

**Bakterizide**:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide**:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin, Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben, Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifosmethyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-transisomer), Cyromazine, DDT, Deltamethrin, Derneton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimethoate, Dimethylvinphos, Dinobuton, Dinocap, Dinetofuran, Diofenolan, Disulfoton, Docusat-sodium, Dofenapyn, DOWCO-439, Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos, Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb, Gamma-HCH, Gossyplure, Grandlure, Granuloseviren, Halfenprox, Halofenozide, HCH, HCN-801, Heptenophos, Hexaflumuron, Hexythiazox, Hydramethylnone, Hydroprene, IKA-2002, Imidacloprid, Imiprothrin, Indoxacarb, Iodofenphos, Iprobenfos, Isazofos, Isofenphos, Isoprocarb, Isoxathion, Ivermectin, Japonilure, Kadethrin, Kernpolyederviren, Kinoprene, Lambda-Cyhalothrin, Lindane, Lufenuron, Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metolcarb, Metoxadiazone, Mevinphos, Milbemectin, Milbemycin, MKI-245, MON-45700, Monocrotophos, Moxidectin, MTI-800, Naled, NC-104, NC-170, NC-184, NC-194, NC-196, Niclosamide, Nicotine, Nitenpyram, Nithiazine,

NNI-0001, NNI-0101, NNI-0250, NNI-9768, Novaluron, Noviflumuron, OK-5101, OK-5201, OK-9601, OK-9602, OK-9701, OK-9802, Omethoate, Oxamyl, Oxydemetonmethyl, Paecilomyces fumosoroseus, Parathion-methyl, Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphosmethyl, Pirimiphos-ethyl, Prallethrin, Profenofos, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen, Quinalphos, Resmethrin, RH-5849, Ribavirin, RU-12457, RU-15525, S-421, S-1833, Salithion, Sebufos, SI-0009, Silafluofen, Spinosad, Spirodiclofen, Spiromesifen, Sulfluramid, Sulfotep, Sulprofos, SZI-121, Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (1R-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Triflumuron, Trimethacarb, Vamidothion, Vaniliprole, Verbutin, Verticillium lecanii, WL-108477, WL-40027, YI-5201, YI-5301, YI-5302, XMC, Xylylcarb, ZA-3274, Zeta-Cypermethrin, Zolaprofos, ZXI-8901, die Verbindung 3-Methylphenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vg1. WO-96/37494, WO-98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden, Düngemitteln, Wachstumsregulatoren, Safenern oder Semiochemicals ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Emteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Ernteprodukte,

höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp.

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.

Aus der Ordnung der Siphonapterida z.B. Pulex spp., Ctenocephalides spp., Xenopsylla spp., Ceratophyllus spp.

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp.

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde,

Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.
Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie Hylotrupes bajulus, Chlorophorus pilosis, Anobium punctatum, Xestobium rufovillosum, Ptilinus pecticomis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittel, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der

Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindelöl und/oder Monochlomaphthalin, vorzugsweise $\alpha$-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher (gemisch) ersetzt werden. Diese Zusätze sollen einer Verflüchtigung der Wirkstoffe sowie einer Kristallisation bzw. Ausfällen vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyfenozide, Triflumuron, Clothianidin, Spinosad, Tefluthrin,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflußkrebse) zusammengefaßt werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis(trialkyl-zinn)-sulfiden, Tri-*n*-butylzinnlaurat, Tri-*n*-butylzinnchlorid, Kupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:

Algizide wie 2-*tert*-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;

Fungizide wie Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie Azaconazole, Cyproconazole, Epoxyconazole, Hexaconazole, Metconazole, Propiconazole und Tebuconazole;

Molluskizide wie Fentinacetat, Metaldehyd, Methiocarb, Niclosamid, Thiodicarb und Trimethacarb, Fe-chelate,

oder herkömmliche Antifouling-Wirkstoffe wie 4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridintriphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, *Chem. Ind.* **1985,** 37, 730-732 und Williams, Antifouling Marine Coatings, Noyes, Park Ridge, **1973** beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfmdungsgemäßen Verbindungen oder die oben genannten Mischungen eingearbeitet werden.

Die Wirkstoffe eignen sich auch zur Bekämpfung von tierischen Schädlingen, insbesondere von Insekten,

Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp.
Aus der Ordnung der Chilopoda z.B. Geophilus spp.
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Phthirus pubis.
Aus der Ordnung der Heteroptera z.B. Cimex hemipterus, Cimex lectularius, Rhodinus prolixus, Triatoma infestans.
Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die Herstellung und die Verwendung der erfindungsgemäßen Wirkstoffe geht aus den folgenden Beispielen hervor.

**Herstellungsbeispiele:**

**Beispiel 1:**

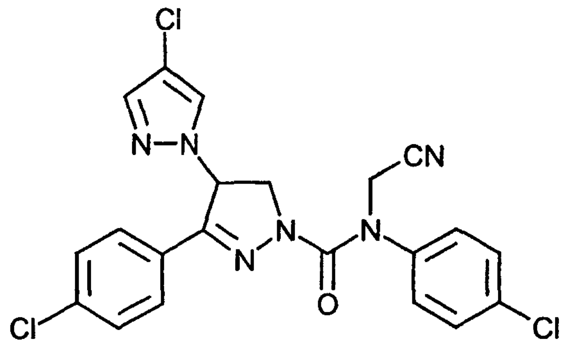

Verfahren (a)

Zu einer Lösung von 16 g (54 mmol) Triphosgen (Kohlensäure-bis-trichlormethylester) in 100 ml Methylenchlorid tropft man bei 0°C eine Lösung von 26,6 g (0,16 mol) N-Cyanomethyl-4-chlor-anilin und 16,5 g (0,16 mol) Triethylamin in 75 ml Methylenchlorid und rührt die Mischung eine halbe Stunde bei Raumtemperatur (ca. 20°C). Dann gibt man eine Lösung von 45,0 g (0,16 Mol) 3-(4-Chlor-phenyl)-4-(4-chlor-pyrazol-1-yl)-4,5-dihydro-1H-pyrazol und 16,5 g (0,16 Mol) Triethylamin in 75 ml Methylenchlorid tropfenweise dazu. Die Reaktionsmischung wird 18 Stunden bei Raumtemperatur nachgerührt und dann zweimal mit je 100 ml Wasser gewaschen. Die organische Phase wird über Natriumsulfat getrocknet und unter vermindertem Druck eingeengt. Den Rückstand verrührt man mit Ethanol, saugt ab und wäscht mit Ethanol nach.

Man erhält 51,5 g (68 % der Theorie) an 3-(4-Chlor-phenyl)-4-(4-chlor-pyrazol-1-yl)-4,5-dihydro-1-pyrazolcarbonsäure-(N-cyanomethyl)-4-chlor-anilid mit dem LogP (pH 2,3) = 3,94.

Beispiel 2:

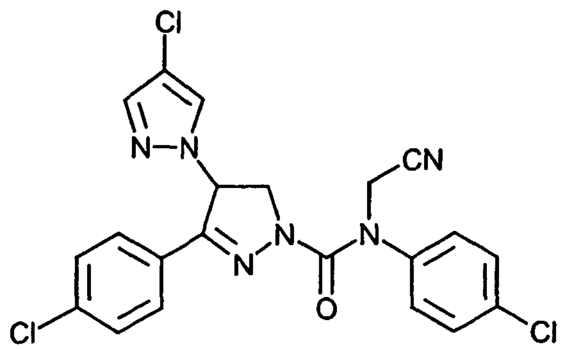

Verfahren (b)

Zu einer Mischung aus 2,81 g (10 mmol) 3-(4-Chlor-phenyl)-4-(4-chlor-pyrazol-1-yl)-4,5-dihydro-1H-pyrazol, 1,5 ml Triethylamin und 50 ml Methylenchlorid gibt man bei 0°C 2,78 g (10 mmol) N-Cyanomethyl-N-(4-chlor-phenyl)-carbamidsäurechlorid. Man rührt das Gemisch 18 Stunden bei Raumtemperatur (ca. 20°C) und wäscht dann zweimal mit je 30 ml Wasser. Die organische Phase wird über Natriumsulfat getrocknet und unter vermindertem Druck eingeengt. Den Rückstand verrührt man mit Ethanol, saugt ab und wäscht mit Ethanol nach.

Man erhält 3,7 g (79 % der Theorie) an 3-(4-Chlor-phenyl)-4-(4-chlor-pyrazol-1-yl)-4,5-dihydro-1-pyrazolcarbonsäure-(N-cyanomethyl)-4-chlor-anilid mit dem LogP (pH 2,3) = 3,94.

Analog zu den Beispielen 1 und 2 bzw. gemäß den allgemeinen Angaben zur Herstellung können auch die in der folgenden Tabelle 1 angegebenen Verbindungen der Formel (I) erhalten werden:

**Tabelle 1:**

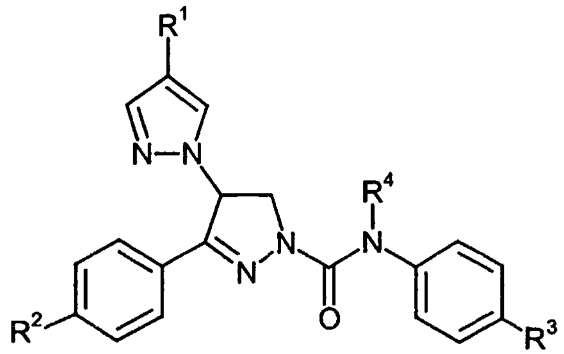

(I)

| Bsp.-Nr. | $R^1$ | $R^2$ | $R^3$ | $R^4$ |
|---|---|---|---|---|
| 3 | Cl | Cl | CN | $CH_2CN$ |
| 4 | Cl | Cl | Br | $CH_2CN$ |
| 5 | Cl | Cl | $SCF_3$ | $CH_2CN$ |
| 6 | Cl | Cl | $OCF_3$ | $CH_2CN$ |
| 7 | Cl | Cl | $CF_3$ | $CH_2CN$ |
| 8 | Cl | Br | Cl | $CH_2CN$ |
| 9 | Cl | Br | CN | $CH_2CN$ |
| 10 | Cl | Br | Br | $CH_2CN$ |
| 11 | Cl | Br | $SCF_3$ | $CH_2CN$ |
| 12 | Cl | Br | $OCF_3$ | $CH_2CN$ |
| 13 | Cl | Br | $CF_3$ | $CH_2CN$ |
| 14 | Cl | F | Cl | $CH_2CN$ |
| 15 | Cl | F | CN | $CH_2CN$ |
| 16 | Cl | F | Br | $CH_2CN$ |
| 17 | Cl | F | $SCF_3$ | $CH_2CN$ |
| 18 | Cl | F | $OCF_3$ | $CH_2CN$ |
| 19 | Cl | F | $CF_3$ | $CH_2CN$ |
| 20 | Br | Cl | CN | $CH_2CN$ |
| 21 | Br | Cl | Br | $CH_2CN$ |
| 22 | Br | Cl | $SCF_3$ | $CH_2CN$ |
| 23 | Br | Cl | $OCF_3$ | $CH_2CN$ |
| 24 | Br | Cl | $CF_3$ | $CH_2CN$ |
| 25 | Br | Br | Cl | $CH_2CN$ |
| 26 | Br | Br | CN | $CH_2CN$ |
| 27 | Br | Br | Br | $CH_2CN$ |
| 28 | Br | Br | $SCF_3$ | $CH_2CN$ |
| 29 | Br | Br | $OCF_3$ | $CH_2CN$ |

Tabelle fortgesetzt

| **Bsp.-Nr.** | **$R^1$** | **$R^2$** | **$R^3$** | **$R^4$** |
|---|---|---|---|---|
| 30 | Br | Br | $CF_3$ | $CH_2CN$ |
| 31 | Br | F | C] | $CH_2CN$ |
| 32 | Br | F | CN | $CH_2CN$ |
| 33 | Br | F | Br | $CH_2CN$ |
| 34 | Br | F | $SCF_3$ | $CH_2CN$ |
| 35 | Br | F | $OCF_3$ | $CH_2CN$ |
| 36 | Br | F | $CF_3$ | $CH_2CN$ |
| 37 | Br | Cl | Cl | $CH_2CN$ |

**Herstellung der Ausganssprodukte der Formel (II)**

Beispiel (II-1):

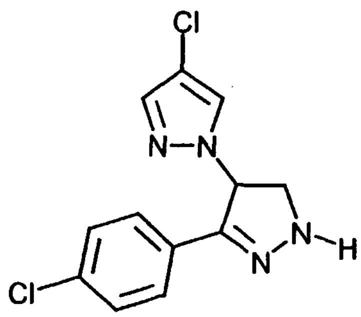

(II-1)

Zu einer Lösung von 5,1 g (0,02 mol) 2-(4-Chlor-pyrazol-1-yl)-4'-chlor-acetophenon in 50 ml Methylenchlorid gibt man bei Raumtemperatur (ca. 20°C) 2,16 g (0,021 mol) Bisdimethylaminomethan und erhitzt die Mischung 18 Stunden unter Rückfluss. Dann destilliert man das Lösungsmittel unter vermindertem Druck ab und löst den Rückstand in 50 ml Ethanol. Nach Zugabe von 1,13 g (0,0226 mol) Hydrazinhydrat wird das Reaktionsgemisch 3 Stunden bei 30°C gerührt. Das ausgefallene Produkt wird abgesaugt, mit etwas kaltem Ethanol und mit Wasser nachgewaschen.

Man erhält 3,42 g (61 % der Theorie) an 3-(4-Chlor-phenyl)-4-(4-chlor-pyrazol-1-yl)-4,5-dihydro-1H-pyrazol mit dem LogP (pH 2,3) = 2,64.

**Herstellung der Ausgangsprodukte der Formel (V)**

Beispiel (V-1)

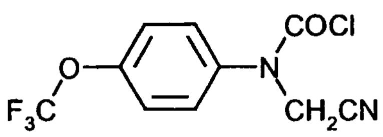

(V-1)

Zu einer Lösung von 10,4 g (0,105 mol) Phosgen in 100 ml Toluol tropft man bei 0°C eine Lösung von 20,7 g (0,0958 mol) N-Cyanomethyl-4-trifluormethoxyanilin und 11,6 g (0,115 mol) Triethylamin in 150 ml Toluol und rührt die Mischung 18 Stunden bei Raumtemperatur (ca. 20°C). Dann wird das überschüssige Phosgen ausgeblasen. Man wäscht

mit Wasser, trocknet die organische Phase über Natriumsulfat und destilliert dann das Lösungsmittel unter vermindertem Druck ab.

Man erhält 26,3 g (94 % der Theorie) N-Cyanomethyl-N-(4-trifluormethoxy)-phenylcarbamidsäurechlorid mit dem Brechungsindex $n_D^{20}$ = 1,4816.

Die Bestimmung der in den Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2.3 mit 0,1 % wässriger Phosphorsäure und Acetonitril als Eluenten; linearer Gradient von 10 % Acetonitril bis 90 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

**Anwendungsbeispiele:**

<u>Beispiel A</u>

Phaedon-Larven-Test

| | |
|---|---|
| Lösungsmittel: | 7 Gewichtsteile Dimethylformamid |
| Emulgator: | 2 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven des Meerrettichblattkäfers (Phaedon cochleariae) besetzt, solange die Blätter noch feucht sind.

Nach der gewünschten Zeit wird die Abtötung in % bestimmt. Dabei bedeutet 100 %, dass alle Käferlarven abgetötet wurden; 0 % bedeutet, dass keine Käferlarven abgetötet wurden.

Bei diesem Test zeigt bei einer beispielhaften Wirkstoffkonzentration von 100 ppm z.B. die Verbindung des Herstellungsbeispiels 1 nach 7 Tagen eine Abtötung von 100 %.

<u>Beispiel B</u>

Plutella-Test

| | |
|---|---|
| Lösungsmittel: | 7 Gewichtsteile Dimethylformamid |
| Emulgator: | 2 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigt bei einer beispielhaften Wirkstoffkonzentration von 100 ppm z.B. die Verbindung des Herstellungsbeispiels 1 nach 7 Tagen eine Abtötung von 100 %.

**Beispiel C**

Spodoptera frugiperda-Test

| | |
|---|---|
| Lösungsmittel: | 7 Gewichtsteile Dimethylformamid |
| Emulgator: | 2 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigt bei einer beispielhaften Wirkstoffkonzentration von 100 ppm z.B. die Verbindung des Herstellungsbeispiels 1 nach 7 Tagen eine Abtötung von 100 %.

**Beispiel D**

**Heliothis armigera-Test**

| | |
|---|---|
| Lösungsmittel: | 7 Gewichtsteile Dimethylformamid |
| Emulgator: | 2 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Sojatriebe (Glycine max) werden durch Tauchen in die Wirkstoffzubereitung der gewünschten Konzentration behandelt und mit Heliothis virescens-Raupen besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigt bei einer beispielhaften Wirkstoffkonzentration von 100 ppm z.B. die Verbindung des Herstellungsbeispiels 1 nach 7 Tagen eine Abtötung von 100 %.

**Beispiel E**

**Diabrotica balteata - Test (**Larven im Boden) Grenzkonzentrations-Test / Bodeninsekten - Behandlung transgener Pflanzen

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,251 Töpfe und lässt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten

gesetzt. Nach weiteren 7 Tagen wird der Wirkungsgrad des Wirkstoffs durch Auszählen der aufgelaufenen Maispflanzen bestimmt (1 Pflanze = 20% Wirkung).

**Beispiel F**

Heliothis virescens - Test (Behandlung transgener Pflanzen)

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

## Patentansprüche

1. Pyrazolincarboxanilide der Formel (I)

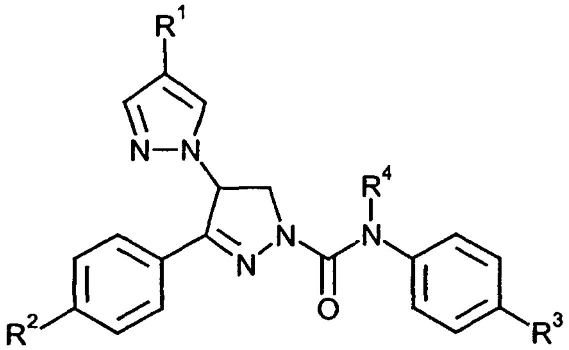

(I)

in welcher

$R^1$ für Halogen steht,
$R^2$ für Cyano, Halogen, Halogenalkyl, Halogenalkoxy, Alkylthio, Halogenalkylthio, Alkylsulfinyl, Halogenalkylsulfinyl, Alkylsulfonyl oder Halogenalkylsulfonyl steht,
$R^3$ für Cyano, Halogen, Halogenalkyl, Halogenalkoxy, Alkylthio, Halogenalkylthio, Alkylsulfinyl, Halogenalkylsulfinyl, Alkylsulfonyl oder Halogenalkylsulfonyl steht und
$R^4$ für Cyanoalkyl steht.

2. Pyrazolincarboxanilide der Formel (I) gemäß Anspruch 1, in welcher

$R^1$ für Chlor, Brom oder Iod steht,
$R^2$ für Cyano, Fluor, Chlor, Brom, Iod, $C_1$-$C_4$-Halogenalkyl, $C_1$-$C_4$-Halogen-alkoxy, $C_1$-$C_4$-Alkylthio, $C_1$-$C_4$-Halogenalkylthio, $C_1$-$C_4$-Alkylsulfinyl, $C_{1}$-$C_4$-Halogenalkylsulfinyl, $C_1$-$C_4$-Alkylsulfonyl oder $C_1$-$C_4$-Halogenalkylsulfonyl steht,
$R^3$ für Cyano, Fluor, Chlor, Brom, Iod, $C_1$-$C_4$-Halogenalkyl, $C_1$-$C_4$-Halogen-alkoxy, $C_1$-$C_4$-Alkylthio, $C_1$-$C_4$-Halogenalkylthio, $C_1$-$C_4$-Alkylsulfinyl, $C_{1}$-$C_4$-Halogenalkylsulfinyl, $C_1$-$C_4$-Alkylsulfonyl oder $C_1$-$C_4$-Halogenalkylsulfonyl steht,

$R^4$ für Cyano-$C_1$-$C_4$-alkyl steht.

3. Pyrazolincarboxanilide der Formel (I) gemäß Anspruch 1, in welcher

$R^1$ für Chlor, Brom oder Iod steht,
$R^2$ für Cyano, Fluor, Chlor, Brom, Iod, Monofluormethyl, Difluormethyl, Trifluormethyl, Monochlormethyl, Dichlormethyl, Trichlormethyl, Chlordifluormethyl, Fluordichlormethyl, Monofluorethyl, Difluorethyl, Trifluorethyl, Tetrafluorethyl, Pentafluorethyl, Monochlorethyl, Dichlorethyl, Trichlorethyl, Chlorfluorethyl, Chlordifluorethyl, Chlortrifluorethyl, Fluordichlorethyl, Dichlordifluorethyl, Difluormethoxy, Trifluormethoxy, Chlordifluormethoxy, Fluorethoxy, Difluorethoxy, Trifluorethoxy, Tetrafluorethoxy, Chlorethoxy, Dichlorethoxy, Chlorfluorethoxy, Methylthio, Ethylthio, n- oder i-Propylthio, Difluormethylthio, Trifluormethylthio, Chlordifluormethylthio, Methylsulfinyl, Ethylsulfinyl, Difluormethylsulfinyl, Trifluormethylsulfinyl, Methylsulfonyl, Ethylsulfonyl, Difluormethylsulfonyl oder Trifluormethylsulfonyl steht,
$R^3$ für Cyano, Fluor, Chlor, Brom, Iod, Monofluormethyl, Difluormethyl, Trichlormethyl, Monochlormethyl, Dichlormethyl, Trichlormethyl, Chlordifluormethyl, Fluordichlormethyl, Monofluorethyl, Difluorethyl, Trifluorethyl, Tetrafluorethyl, Pentafluorethyl, Monochlorethyl, Dichlorethyl, Trichlorethyl, Chlorfluorethyl, Chlordifluorethyl, Chlortrifluorethyl, Fluordichlorethyl, Dichlordifluorethyl, Difluormethoxy, Trifluormethoxy, Chlordifluormethoxy, Fluorethoxy, Difluorethoxy, Trifluorethoxy, Tetrafluorethoxy, Chlorethoxy, Dichlorethoxy, Chlorfluorethoxy, Methylthio, Ethylthio, n- oder i-Propylthio, Difluormethylthio, Trifluormethylthio, Chlordifluormethylthio, Methylsulfinyl, Ethylsulfinyl, Difluorrnethylsulfinyl, Trifluormethylsulfinyl, Methylsulfonyl, Ethylsulfonyl, Difluormethylsulfonyl oder Trifluormethylsulfonyl steht,
$R^4$ für Cyanomethyl, Cyanoethyl oder Cyanopropyl steht.

4. Pyrazolincarboxanilide der Formel (I) gemäß Anspruch 1, in welcher

$R^1$ für Chlor oder Brom steht,
$R^2$ für Cyano, Fluor, Chlor, Brom, Iod, Difluormethyl, Trifluormethyl, Dichlormethyl, Trichlormethyl, Chlordifluormethyl, Fluordichlormethyl, Difluormethoxy, Trifluormethoxy, Chlordifluormethoxy, Difluormethylthio, Trifluormethylthio, Chlordifluormethylthio, Trifluormethylsulfinyl oder Trifluormethylsulfonyl steht,
$R^3$ für Cyano, Fluor, Chlor, Brom, Iod, Difluormethyl, Trifluormethyl, Dichlormethyl, Trichlormethyl, Chlordifluormethyl, Fluordichlormethyl, Difluormethoxy, Trifluormethoxy, Chlordifluormethoxy, Difluormethylthio, Trifluormethylthio, Chlordifluormethylthio, Trifluormethylsulfinyl oder Trifluormethylsulfonyl steht,
$R^4$ für Cyanomethyl steht.

5. Verfahren zum Herstellen von Pyrazolincarboxaniliden der Formel (I) gemäß Anspruch 1, **dadurch gekennzeichnet, dass** man

(a) substituierte Pyrazoline der Formel (II)

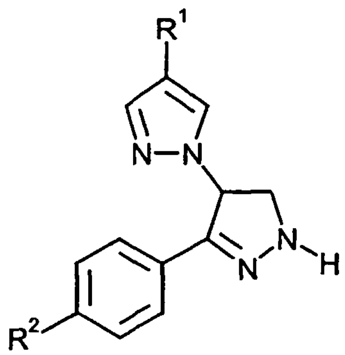

(II)

in welcher $R^1$ und $R^2$ die in Anspruch 1 angegebenen Bedeutungen haben,
mit Isocyanaten der Formel (III)

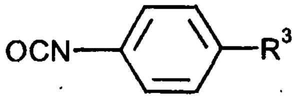

(III)

in welcher $R^3$ die in Anspruch 1 angegebenen Bedeutungen hat, gegebenenfalls in Gegenwart eines Verdünnungsmittels und gegebenenfalls in Gegenwart eines Katalysators umsetzt und die so erhaltenen substituierten Pyrazolincarboxanilide der Formel (Ia)

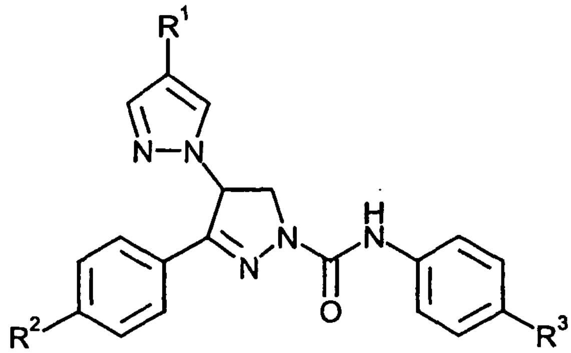

(Ia)

in welcher
$R^1$, $R^2$ und $R^3$ die in Anspruch 1 angegebenen Bedeutungen haben, mit Halogenverbindungen der Formel (IV)

$X^1—R^4$ (IV)

in welcher

$R^4$ die in Anspruch 1 angegebene Bedeutung hat und
$X^1$ für Halogen steht,

gegebenenfalls in Gegenwart eines Verdünnungsmittels und gegebenenfalls in Gegenwart einer Base umsetzt, oder dass man die substituierten Pyrazolincarboxanilide der Formel (Ib)

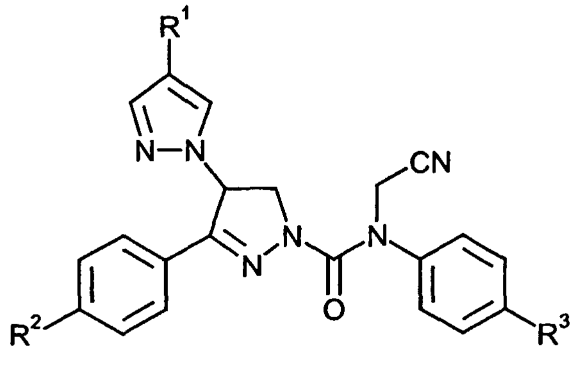

(Ib)

in welcher $R^1$, $R^2$ und $R^3$ die in Anspruch 1 angegebenen Bedeutungen haben, erhält, indem man
(b) substituierte Pyrazoline der Formel (II)

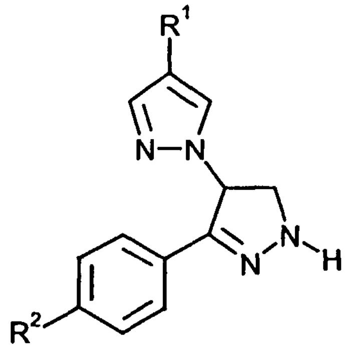

(II)

in welcher $R^1$ und $R^2$ die in Anspruch 1 angegebenen Bedeutungen haben, mit Carbamidsäurechloriden der Formel (V)

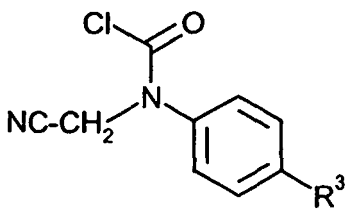

(V)

in welcher $R^3$ die in Anspruch 1 angegebenen Bedeutungen hat, in Gegenwart eines Verdünnungsmittels und gegebenenfalls in Gegenwart einer Base umsetzt.

**6.** Schädlingsbekämpfungsmittel, **gekennzeichnet durch** einen Gehalt an mindestens einer Verbindung der Formel (I) gemäß Anspruch 1 neben Streckmitteln und/oder oberflächenaktiven Stoffen.

**7.** Nichttherapeutische Verwendung von Verbindungen der Formel (I) gemäß Anspruch 1 zur Bekämpfung von Schädlingen.

**8.** Verfahren zur nichttherapeutischen Bekämpfung von Schädlingen, **dadurch gekennzeichnet, dass** man Verbindungen der Formel (I) gemäß Anspruch 1 auf Schädlinge und/oder ihren Lebensraum einwirken lässt.

**9.** Verfahren zur Herstellung von Schädlingsbekämpfungsmitteln, **dadurch gekennzeichnet, dass** man Verbindungen der Formel (I) gemäß Anspruch 1 mit Streckmitteln und/oder oberflächenaktiven Stoffen vermischt.

**Claims**

**1.** Pyrazolinecarboxanilides of the formula (I)

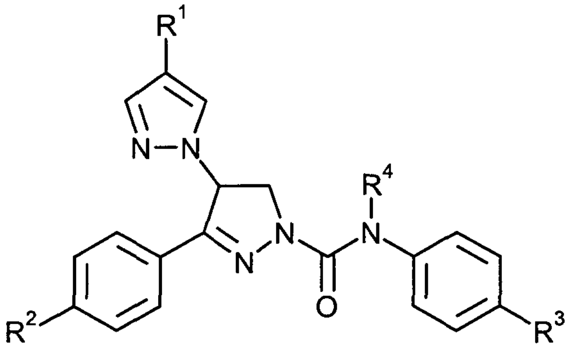

(I)

in which

$R^1$ represents halogen,
$R^2$ represents cyano, halogen, haloalkyl, haloalkoxy, alkylthio, haloalkylthio, alkylsulphinyl, haloalkylsulphinyl, alkylsulphonyl or haloalkylsulphonyl,
$R^3$ represents cyano, halogen, haloalkyl, haloalkoxy, alkylthio, haloalkylthio, alkylsulphinyl, haloalkylsulphinyl, alkylsulphonyl or haloalkylsulphonyl and
$R^4$ represents cyanoalkyl.

**2.** Pyrazolinecarboxanilides of the formula (I) according to Claim 1 in which

$R^1$ represents chlorine, bromine or iodine,
$R^2$ represents cyano, fluorine, chlorine, bromine, iodine, $C_1$-$C_4$-haloalkyl, $C_1$-$C_4$-haloalkoxy, $C_1$-$C_4$-alkylthio, $C_1$-$C_4$-haloalkylthio, $C_1$-$C_4$-alkylsulphinyl, $C_1$-$C_4$-haloalkylsulphinyl, $C_1$-$C_4$-alkylsulphonyl or $C_1$-$C_4$-haloalkylsulphonyl,
$R^3$ represents cyano, fluorine, chlorine, bromine, iodine, $C_1$-$C_4$-haloalkyl, $C_1$-$C_4$-haloalkoxy, $C_1$-$C_4$-alkylthio, $C_1$-$C_4$-haloalkylthio, $C_1$-$C_4$-alkylsulphinyl, $C_1$-$C_4$-haloalkylsulphinyl, $C_1$-$C_4$-alkylsulphonyl or $C_1$-$C_4$-haloalkylsulphonyl,
$R^4$ represents cyano-$C_1$-$C_4$-alkyl.

**3.** Pyrazolinecarboxanilides of the formula (I) according to Claim 1 in which

$R^1$ represents chlorine, bromine or iodine,
$R^2$ represents cyano, fluorine, chlorine, bromine, iodine, monofluoromethyl, difluoromethyl, trifluoromethyl, monochloromethyl, dichloromethyl, trichloromethyl, chlorodifluoromethyl, fluorodichloromethyl, monofluoroethyl, difluoroethyl, trifluoroethyl, tetrafluoroethyl, pentafluoroethyl, monochloroethyl, dichloroethyl, trichloroethyl, chlorofluoroethyl, chlorodifluoroethyl, chlorotrifluoroethyl, fluorodichloroethyl, dichlorodifluoroethyl, difluoromethoxy, trifluoromethoxy, chlorodifluoromethoxy, fluoroethoxy, difluoroethoxy, trifluoroethoxy, tetrafluoroethoxy, chloroethoxy, dichloroethoxy, chlorofluoroethoxy, methylthio, ethylthio, n-or i-propylthio, difluoromethylthio, trifluoromethylthio, chlorodifluoromethylthio, methylsulphinyl, ethylsulphinyl, difluoromethylsulphinyl, trifluoromethylsulphinyl, methylsulphonyl, ethylsulphonyl, difluoromethylsulphonyl or trifluoromethylsulphonyl,
$R^3$ represents cyano, fluorine, chlorine, bromine, iodine, monofluoromethyl, difluoromethyl, trifluoromethyl, monochloromethyl, dichloromethyl, trichloromethyl, chlorodifluoromethyl, fluorodichloromethyl, monofluoroethyl, difluoroethyl, trifluoroethyl, tetrafluoroethyl, pentafluoroethyl, monochloroethyl, dichloroethyl, trichloroethyl, chlorofluoroethyl, chlorodifluoroethyl, chlorotrifluoroethyl, fluorodichloroethyl, dichlorodifluoroethyl, difluoromethoxy, trifluoromethoxy, chlorodifluoromethoxy, fluoroethoxy, difluoroethoxy, trifluoroethoxy, tetrafluoroethoxy, chloroethoxy, dichloroethoxy, chlorofluoroethoxy, methylthio, ethylthio, n-or i-propylthio, difluoromethylthio, trifluoromethylthio, chlorodifluoromethylthio, methylsulphinyl, ethylsulphinyl, difluoromethylsulphinyl, trifluoromethylsulphinyl, methylsulphonyl, ethylsulphonyl, difluoromethylsulphonyl or trifluoromethylsulphonyl,
$R^4$ represents cyanomethyl, cyanoethyl or cyanopropyl.

**4.** Pyrazolinecarboxanilides of the formula (I) according to Claim 1 in which

$R^1$ represents chlorine or bromine,
$R^2$ represents cyano, fluorine, chlorine, bromine, iodine, difluoromethyl, trifluoromethyl, dichloromethyl, trichloromethyl, chlorodifluoromethyl, fluorodichloromethyl, difluoromethoxy, trifluoromethoxy, chlorodifluoromethoxy, difluoromethylthio, trifluoromethylthio, chlorodifluoromethylthio, trifluoromethylsulphinyl or trifluoromethylsulphonyl,

$R^3$ represents cyano, fluorine, chlorine, bromine, iodine, difluoromethyl, trifluoromethyl, dichloromethyl, trichloromethyl, chlorodifluoromethyl, fluorodichloromethyl, difluoromethoxy, trifluoromethoxy, chlorodifluoromethoxy, difluoromethylthio, trifluoromethylthio, chlorodifluoromethylthio, trifluoromethylsulphinyl or trifluoromethylsulphonyl,

$R^4$ represents cyanomethyl.

**5.** Process for preparing pyrazolinecarboxanilides of the formula (I) according to Claim 1, **characterized in that**

(a) substituted pyrazolines of the formula (II)

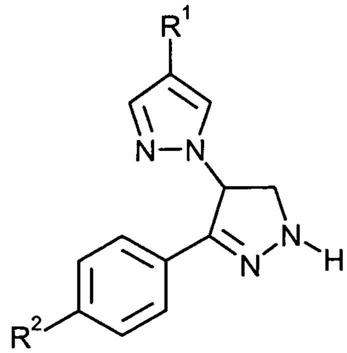

(II)

in which $R^1$ and $R^2$ are as defined in Claim 1,
are reacted with isocyanates of the formula (III)

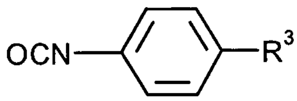

(III)

in which $R^3$ is as defined in Claim 1,
if appropriate in the presence of a diluent and if appropriate in the presence of a catalyst, and the resulting substituted pyrazolinecarboxanilides of the formula (Ia)

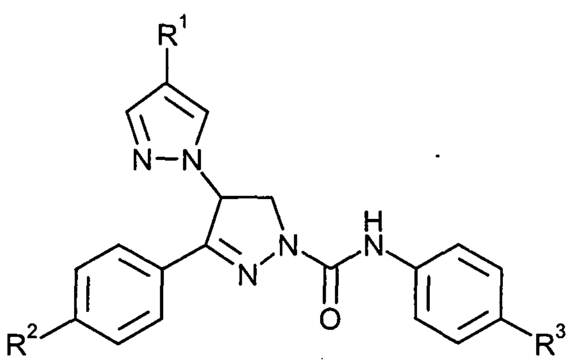

(Ia)

in which
$R^1$, $R^2$ and $R^3$ are as defined in Claim 1,
are reacted with halogen compounds of the formula (IV)

$X^1$-$R^4$ (IV)

in which

$R^4$ is as defined in Claim 1 and
$X^1$ represents halogen,

if appropriate in the presence of a diluent and if appropriate in the presence of a base, or that the substituted pyrazolinecarboxanilides of the formula (Ib)

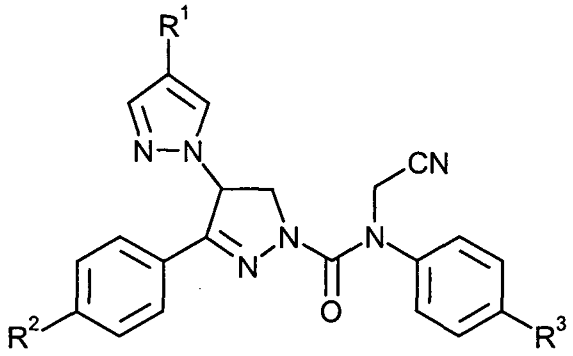

(Ib)

in which R', $R^2$ and $R^3$ are as defined in Claim 1 are obtained by
(b) reacting substituted pyrazolines of the formula (II)

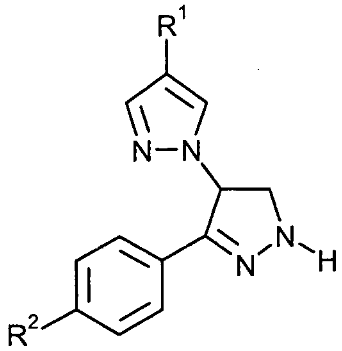

(II)

in which $R^1$ and $R^2$ are as defined in Claim 1 with carbamoyl chlorides of the formula (V)

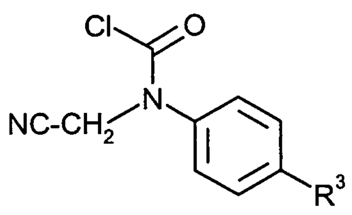

(V)

in which $R^3$ is as defined in Claim 1 in the presence of a diluent and if appropriate in the presence of a base.

**6.** Pesticides, **characterized in that** they comprise at least one compound of the formula (I) according to Claim 1, in

addition to extenders and/or surfactants.

7. Non-therapeutic use of compounds of the formula (I) according to Claim 1 for controlling pests.

8. Method for the non-therapeutic control of pests, **characterized in that** compounds of the formula (I) according to Claim 1 are allowed to act on pests and/or their habitat.

9. Process for preparing pesticides, **characterized in that** compounds of the formula (I) according to Claim 1 are mixed with extenders and/or surfactants.

## Revendications

1. Pyrazoline-carboxanilides de formule (I)

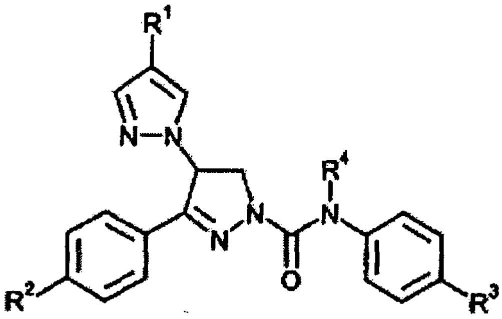

(I)

dans laquelle

$R^1$ représente un halogène,
$R^2$ représente un groupe cyano, un halogène, un reste halogénalkyle, halogénalkoxy, alkylthio, halogénalkylthio, alkylsulfinyle, halogénalkylsulfinyle, alkylsulfonyle ou halogénalkylsulfonyle,
$R^3$ représente un groupe cyano, un halogène, un reste halogénalkyle, halogénalkoxy, alkylthio, halogénalkylthio, alkylsulfinyle, halogénalkylsulfinyle, alkylsulfonyle ou halogénalkylsulfonyle et
$R^4$ est un reste cyanalkyle.

2. Pyrazoline-carboxanilides de formule (I) suivant la revendication 1, formule dans laquelle

$R^1$ représente le chlore, le brome ou l'iode,
$R^2$ représente un groupe cyano, le fluor, le chlore, le brome, l'iode, un reste halogénalkyle en $C_1$ à $C_4$, halogénalkoxy en $C_1$ à $C_4$, alkylthio en $C_1$ à $C_4$, halogénalkylthio en $C_1$ à $C_4$, alkylsulfinyle en $C_1$ à $C_4$, halogénalkylsulfinyle en $C_1$ à $C_4$, alkylsulfonyle en $C_1$ à $C_4$ ou halogénalkylsulfonyle en $C_1$ à $C_4$,
$R^3$ représente un groupe cyano, le fluor, le chlore, le brome, l'iode, un reste halogénalkylke en $C_1$ à $C_4$, halogénalkoxy en $C_1$ à $C_4$, alkylthio en $C_1$ à $C_4$, halogénalkylthio en $C_1$ à $C_4$, alkylsulfinyle en $C_1$ à $C_4$, halogénalkylsulfinyle en $C_1$ à $C_4$, alkylsulfonyle en $C_1$ à $C_4$ ou halogénalkylsulfonyle en $C_1$ à $C_4$,
$R^4$ est un reste cyano-(alkyle en $C_1$ à $C_4$) .

3. Pyrazoline-carboxanilides de formule (I) suivant la revendication 1, formule dans laquelle

$R^1$ représente le chlore, le brome ou l'iode,
$R^2$ représente un groupe cyano, le fluor, le chlore, le brome, l'iode, un reste monofluorométhyle, difluorométhyle, trifluorométhyle, monochlorométhyle, dichlorométhyle, trichlorométhyle, chlorodifluorométhyle, fluorodichlorométhyle, monofluoréthyle, difluoréthyle, trifluoréthyle, tétrafluoréthyle, pentafluoréthyle, monochloréthyle, dichloréthyle, trichloréthyle, chlorofluoréthyle, chlorodifluoréthyle, chlorotrifluoréthyle, fluorodichloréthyle, dichlorodifluoréthyle, difluorométhoxy, trifluorométhoxy, chlorodifluorométhoxy, fluoréthoxy, difluoréthoxy, trifluoréthoxy, tétrafluoréthoxy, chloréthoxy, dichloréthoxy, chlorofluoréthoxy, méthylthio, éthylthio, n-propylthio, isopropylthio, difluorométhylthio, trifluorométhylthio, chlorodifluorométhylthio, méthylsulfinyle, éthylsulfinyle, difluorométhylsulfinyle, trifluorométhylsulfinyle, méthylsulfonyle, éthylsulfonyle, difluorométhylsulfonyle ou trifluorométhylsulfonyle

thylsulfonyle,

$R^3$ représente un groupe cyano, le fluor, le chlore, le brome, l'iode, un reste monofluorométhyle, difluorométhyle, trichlorométhyle, monochlorométhyle, dichlorométhyle, trichlorométhyle, chlorodifluorométhyle, fluorodichlorométhyle, monofluoréthyle, difluoréthyle, trifluoréthyle, tétrafluoréthyle, pentafluoréthyle, monochloréthyle, dichloréthyle, trichloréthyle, chlorofluoréthyle, chlorodifluoréthyle, chlorotrifluoréthyle, fluorodichloréthyle, dichlorodifluoréthyle, difluorométhoxy, trifluorométhoxy, chlorodifluorométhoxy, fluoréthoxy, difluoréthoxy, trifluoréthoxy, tétrafluoréthoxy, chloréthoxy, dichloréthoxy, chlorofluoréthoxy, méthylthio, éthylthio, n-propylthio, isopropylthio, difluorométhylthio, trifluorométhylthio, chlorodifluorométhylthio, méthylsulfinyle, éthylsulfinyle, difluorométhylsulfinyle, trifluorométhylsulfinyle, méthylsulfonyle, éthylsulfonyle, difluorométhylsulfonyle ou trifluorométhylsulfonyle,

$R^4$ représente un reste cyanométhyle, cyanéthyle ou cyanopropyle.

**4.** Pyrazoline-carboxanilides de formule (I) suivant la revendication 1, formule dans laquelle

$R^1$ représente le chlore ou le brome,

$R^2$ représente un groupe cyano, le fluor, le chlore, le brome, l'iode, un reste difluorométhyle, trifluorométhyle, dichlorométhyle, trichlorométhyle, chlorodifluorométhyle, fluorodichlorométhyle, difluorométhoxy, trifluorométhoxy, chlorodifluorométhoxy, difluorométhylthio, trifluorométhylthio, chlorodifluorométhylthio, trifluorométhylsulfinyle ou trifluorométhylsulfonyle,

$R^3$ représente un groupe cyano, le fluor, le chlore, le brome, l' iode, un reste difluorométhyle, trifluorométhyle, dichlorométhyle, trichlorométhyle, chlorodifluorométhyle, fluorodichlorométhyle, difluorométhoxy, trifluorométhoxy, chlorodifluorométhoxy, difluorométhylthio, trifluorométhylthio, chlorodifluorométhylthio, trifluorométhylsulfinyle ou trifluorométhylsulfonyle,

$R^4$ représente un reste cyanométhyle.

**5.** Procédé de production de pyrazoline-carboxanilides de formule (I) suivant la revendication 1, **caractérisé en ce que**

(a) on fait réagir des pyrazolines substituées de formule (II)

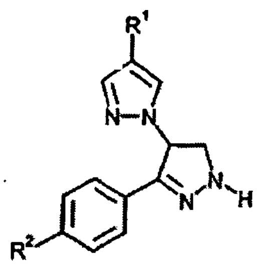

(II)

dans laquelle $R^1$ et $R^2$ ont les définitions indiquées dans la revendication 1,
avec des isocyanates de formule (III)

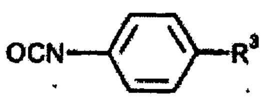

(III)

dans laquelle $R^3$ a les définitions indiquées dans la revendication 1,
éventuellement en présence d'un diluant et, le cas échéant, en présence d'un catalyseur et on fait réagir les pyrazoline-carboxanilides substitués ainsi obtenus de formule (Ia)

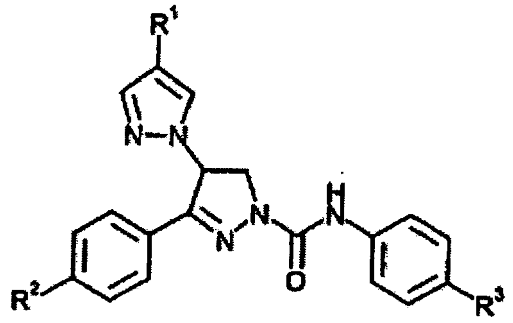

(Ia)

dans laquelle
$R^1$, $R^2$ et $R^3$ ont les définitions indiquées dans la revendication 1,
avec des composés halogénés de formule (IV)

$X^1$-$R^4$ (IV)

dans laquelle

$R^4$ a la définition indiquée dans la revendication 1 et
$X^1$ représente un halogène,
éventuellement en présence d'un diluant et, le cas échéant, en présence d'une base,
ou bien on obtient les pyrazoline-carboxanilides substitués de formule (Ib)

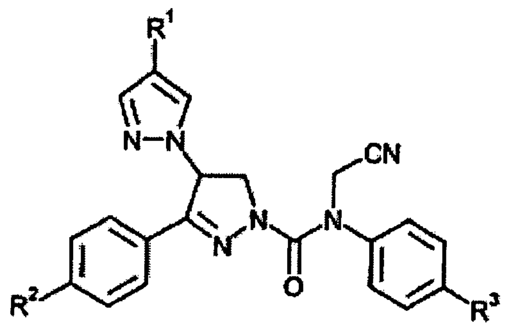

(Ib)

dans laquelle $R^1$, $R^2$ et $R^3$ ont les définitions indiquées dans la revendication 1,
(b) en faisant réagir des pyrazolines substituées de formule (II)

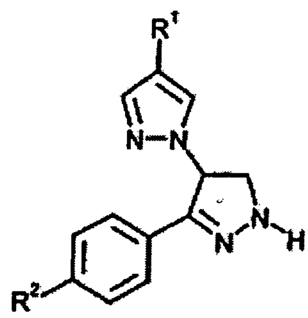

(II)

dans laquelle $R^1$ et $R^2$ ont les définitions indiquées dans la revendication 1,
avec des chlorures d'acides carbamiques de formule (V)

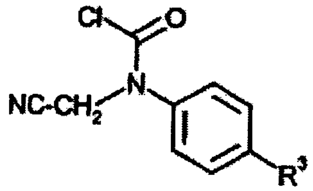

(V)

dans laquelle $R^3$ a les définitions indiquées dans la revendication 1, en présence d'un diluant et, le cas échéant, en présence d'une base.

6. Composition pesticide, **caractérisée par** une teneur en au moins un composé de formule (I) suivant la revendication 1, à côté de diluants et/ou d'agents tensioactifs.

7. Utilisation non thérapeutique de composés de formule (I) suivant la revendication 1 pour combattre des parasites.

8. Procédé de lutte non thérapeutique contre des parasites, **caractérisé en ce qu'**on fait agir des composés de formule (I) suivant la revendication 1 sur des parasites et/ou sur leur milieu.

9. Procédé de préparation de compositions pesticides, **caractérisé en ce qu'**on mélange des composés de formule (I) suivant la revendication 1 avec des diluants et/ou des agents tensioactifs.